# EUROPEAN PATENT SPECIFICATION

(11) **EP 2 121 931 B1**
(45) Date of publication and mention of the grant of the patent: **11.05.2011**
(21) Application number: 08708212.9
(22) Date of filing: 25.01.2008
(51) Int. Cl.: C12N 15/62, C07K 14/705, C07K 1/22, A61K 38/04

(54) **PURIFICATION OF FC-TACT FUSION PROTEINS USING THE OILBODY TECHNOLOGY**
AUFREINIGUNG VON FC-TACT-FUSIONSPROTEINEN UNTER VERWENDUNG DER ÖLKÖRPER-TECHNIK
PURIFICATION DE PROTÉINES HYBRIDES FC-TACT UTILISANT LA TECHNOLOGIE À BASE DE CORPS HUILEUX

(30) Priority: 26.01.2007 EP 07101233; 26.01.2007 US 886681 P
(43) Date of publication of application: 25.11.2009
(73) Proprietor: Merck Serono S.A., 1267 Coinsins (CH)
(72) Inventor: KORNMANN, Henri, CH-1290 Versoix (CH); BAER, Gianni, CH-1814 La Tour-de-Peilz (CH)
(74) Representative: Merck Serono SA - Geneva Intellectual Property
(86) International application number: PCT/EP2008/050886
(87) International publication number: WO 2008/090217

(56) References cited:
- WO-A-00/40716
- WO-A-98/27115
- WO-A-02/094852
- G-U VON BÜLOW & R J BRAM: "NF-AT activation induced by a CAML-interacting member of the tumor necrosis factor receptor superfamily" SCIENCE., vol. 278, 2 October 1997 (1997-10-02), pages 138-141, XP002140938 US AMERICAN ASSOCIATION FOR THE ADVANCEMENT OF SCIENCE, WASHINGTON, DC. cited in the application

## Description

### FIELD OF THE INVENTION

The present invention is in the field of protein purification. More specifically, it relates to the purification of TACI-Fc using genetically engineered oilbodies expressing protein A.

### BACKGROUND OF THE INVENTION

### 1. Fc-fusion proteins

Fc-fusion proteins are chimeric proteins consisting of an effector region of a protein, such as e.g. the binding region of a receptor, fused to the Fc region of an immunoglobulin that is frequently an immunoglobulin G (IgG). Fc-fusion proteins are widely used as therapeuticals as they offer advantages conferred by the Fc region, such as:
- the possibility of purification using protein A or protein G affinity chromatography with affinities that vary accordingly to the IgG isotype. Human IgG₁, IgG₂ and IgG₄ bind strongly to Protein A and all human IgGs including IgG₃ bind strongly to Protein G; and
- an increased half-life in the circulatory system, since the Fc region binds to the salvage receptor FcRn which protects from lysosomal degradation.

Serum half-life and effector functions can be modulated by engineering the Fc region to increase or reduce its binding to FcRn, FcγRs and C1q respectively, depending on the therapeutic use intended for the Fc-fusion protein.

In ADCC, the Fc region of an antibody binds to Fc receptors (FcγRs) on the surface of immune effector cells such as natural killers and macrophages, leading to the phagocytosis or lysis of the targeted cells.

In CDC, the antibodies kill the targeted cells by triggering the complement cascade at the cell surface. IgG isoforms exert different levels of effector functions increasing in the order of IgG₄ < IgG₂ < IgG₁ ≤ IgG₃. Human IgG₁ displays high ADCC and CDC, and is the most suitable for therapeutic use against pathogens and cancer cells.

Modifying effector functions can be achieved by engineering the Fc region to either improve or reduce their binding to FcγRs or the complement factors.

The binding of IgG to the activating (FcγRI, FcγRIIa, FcγRIIIa and FcγRIIIb) and inhibitory (FcγRIIb) FcγRs or the first component of complement (C1q) depends on residues located in the hinge region and the CH2 domain. Two regions of the CH2 domain are critical for FcγRs and complement C1q binding, and have unique sequences in IgG₂ and IgG₄. For instance, substitution of IgG₂ residues at positions 233-236 into human IgG₁ greatly reduced ADCC and CDC (Armour et al., 1999; Shields et al., 2001)

Numerous mutations have been made in the CH2 domain of IgG and their effect on ADCC and CDC was tested *in vitro* (Shields et al., 2001; Steurer et al., 1995). In particular, a mutation to alanine at E333 was reported to increase both ADCC and CDC (Idusogie et al., 2000; Idusogie et al., 2001).

Increasing the serum half-life of a therapeutic Fc-fusion protein is another way to improve its efficacy, allowing higher circulating levels, less frequent administration and reduced doses. This can be achieved by enhancing the binding of the Fc region to neonatal FcR (FcRn). FcRn, which is expressed on the surface of endothelial cells, binds the IgG in a pH-dependent manner and protects it from degradation. Several mutations located at the interface between the CH2 and CH3 domains have been shown to increase the half-life of IgG₁ (Hinton et al., 2004; Vaccaro et al., 2005).

The following Table 1 summarizes some known mutations of the IgG Fc-region (taken from Invivogen's website).

**Table 1**

| **Engineered Fc** | **IgG Isotype** | **Mutations** | **Properties** | **Potential Benefits** | **Applications** |
|---|---|---|---|---|---|
| hlgG1e1 | human IgG1 | T250Q/M428L | Increased plasma half-life | Improved localization to target; increased efficacy; reduced dose or frequency of administration | Vaccination; therapeutic use |
| hlgG1e2 | human IgG1 | M252Y/S254T/T2 56E + H433K/N434F | Increased plasma half-life | Improved localization to target; increased efficacy; reduced dose or frequency of administration | Vaccination; therapeutic use |
| hlgG1e3 | human IgG1 | E233P/L234V/L23 5A/ΔG236 + A327G/A330S/P3 31S | Reduced ADCC and CDC | Reduced adverse events | Therapeutic use without cell depletion |
| hlgG1e4 | human IgG1 | E333A | Increased ADCC and CDC | Increased efficacy | Therapeutic use with cell depletion |
| hlgG2e1 | human IgG2 | K322A | Reduced CDC | Reduced adverse events | Vaccination; therapeutic use |

### 2. Purification of Fc-fusion proteins

Fc-fusion proteins have become commercially important as drugs that are generally called "biologicals". One of the greatest challenges is the development of cost effective and efficient processes for purification of proteins on a commercial scale. While many methods are now available for large-scale preparation of proteins, crude products, such as cell culture supernatants, contain not only the desired product but also impurities, which are difficult to separate from the desired product. Although cell culture supernatants of cells expressing recombinant protein products may contain less impurities if the cells are grown in serum-free medium, the host cell proteins (HCPs) still remain to be eliminated during the purification process. Additionally, the health authorities request high standards of purity for proteins intended for human administration.

Many purification methods contain steps requiring application of low or high pH, high salt concentrations or other extreme conditions that may jeopardize the biological activity of a given protein. Thus, for any protein it is a challenge to establish a purification process allowing for sufficient purity while maintaining the productivity of the process in terms of price per quantity of produced protein.

The purification of Fc-fusion proteins is usually based on the affinity of the Fc-fusion protein to another protein that is immobilized to a chromatography resin. Examples for such immobilized ligands are the bacterial cell wall proteins Protein A and Protein G, having specificity to the Fc portion of certain immunoglobulins. Although both Protein A and Protein G have a strong affinity for IgG antibodies, they have varying affinities to other immunoglobulin classes and isotypes as well.

Protein A is a 43,000 Dalton protein that is produced by the bacteria *Staphylococcus aureus* and contains four binding sites to the Fc regions of IgG. Protein G is produced from group G Streptococci and has two binding sites for the IgG Fc region. Both proteins have been widely characterized for their affinity to various types of immunoglobulins. Protein L is a further bacterial protein, originating from Peptostreptococcus, binding to Immunoglobulins and fragments thereof containing Ig light chains (Akerstrom and Bjorck, 1989).

Protein A, Protein G and Protein L affinity chromatography are widely used for isolation and purification of immunoglobulins. Since the binding sites for Protein A and Protein G reside in the Fc region of an immunoglobulin, Protein A and Protein G affinity chromatography also allows purification of so-called Fc-fusion proteins. However, purification involving Protein A, Protein G and Protein L affinity chromatography are both expensive and time-consuming procedures.

Therefore, there is an unmet need for an efficient, easy and cost-effective purification method for Fc-fusion proteins.

### 3. The oilbody technology

U.S. patent No. 6,924,363 (Moloney et al., 2005) and PCT publication WO 98/27115 disclose a technology referred to as the "oilbody technology". This technology is based on the use of oilbodies and their associated proteins as affinity matrices for the separation and purification of target molecules. One of the examples of U.S. patent No. 6,924,363 and WO 98/27115 is a proof of concept showing that a labeled rabbit anti-mouse antibody can be attached and also eluted from genetically engineered oilbodies expressing Protein A. However, U.S. patent No. 6,924,363 and WO 98/27115 do not teach any industrial purification process of a therapeutic protein. In addition, while U.S. patent No. 6,924,363 and WO 98/27115 mention antibodies, they does not suggest the use of the oilbody technology for the separation and/or purification of Fc-fusion proteins. They do not teach the optimal conditions for purifying antibodies or Fc-fusion proteins, either.

### 4. TACI-Fc fusion proteins

The transmembrane activator and calcium-modulator and cyclophilin ligand interactor, referred to as TACI, is a member of the TNF-R superfamily. TACI is a transmembrane activator and CAML-interactor (von Bulow and Bram, 1997; Gross et al., 2000), which has an extracellular domain containing two cysteine-rich pseudo-repeats. TACI binds two members of the tumor necrosis factor (TNF) ligand family. One ligand is designated BLyS, BAFF, neutrokine-α, TALL-1, zTNF4, or THANK (Moore et al., 1999). The other ligand has been designated as APRIL, TNRF death ligand-1 or ZTNF2 (Hahne et al., 1998).

Fusion proteins containing soluble forms of the TACI receptor fused to an IgG Fc region are known as well and were designated TACI-Fc (WO 00/40716, WO 02/094852). TACI-Fc inhibits the binding of BLyS and APRIL to B-cells (Xia et al., 2000). It is being developed for the treatment autoimmune diseases, including systemic lupus erythematosus (SLE), rheumatoid arthritis (RA) and hematological malignancies, as well as for treatment of multiple sclerosis (MS). In addition to this, TACI-Fc is being developed in multiple myeloma (MM) (Novak et al., 2004) and non-Hodgkin's lymphoma (NHL), chronic lymphocytic leukemia (CLL) and Waldenstrom's macroglobulemia (WM). The molecule referred to as atacicept, which is currently in clinical trials, is for example a TACI-Fc fusion protein.

TACI-Fc is active as a dimer comprised of two TACI-Fc proteins. When producing TACI-Fc, one also obtains aggregates comprised of two or more dimers. The purification process of TACI-Fc should thus allow to reduce the percentage of TACI-Fc aggregates.

The methods for purifying TACI-Fc that are available in the art involve a Protein A chromatography. For example, Wu et al. (2000) discloses the purification of the TACI-Fc fusion protein by Protein A chromtagraphy, the chromatography column being loaded with the HyperD^{®} Solvent-Detergent Removal Chromatography Resin.

Given the therapeutic utility of TACI-Fc, there is a need for obtaining significant amounts of purified TACI-Fc dimers using an easy and cost-effective process.

### SUMMARY OF THE INVENTION

The present invention is based on the development of a purification process for TACI-Fc based on the oilbody technology.

Therefore, a first aspect of the present invention is directed to a method for purifying a TACI-Fc protein comprising the steps of:
a) mixing a solution comprising protein A oilbodies with a sample comprising said TACI-Fc protein in such a way as to obtain a final ratio of mg of TACI-Fc protein per mg of dry-weight oilbodies within a range of about 1 to about 5.5; and
b) separating said protein A oilbodies from said TACI-Fc protein by adding an aqueous solution having a pH comprised within a range of 3.5 to 3.9,
wherein the solution comprising TACI-Fc obtained at the end of step (b) comprises more than 93%, 94%, 95% or 96% of TACI-Fc dimers.

A second aspect of the invention is directed to a method of manufacturing a pharmaceutical composition comprising TACI-Fc comprising the steps of:
a) purifying said TACI-Fc according to the method of the invention; and
b) formulating said purified TACI-Fc into a pharmaceutical composition.

The use of protein A oilbodies for purifying TACI-Fc is described.

### BRIEF DESCRIPTION OF THE FIGURES

Figure 1A is a scheme of an oilbody taken from the website of SemBioSys Genetics Inc.
Figure 1B is a schematic diagram illustrating a protein A oilbody expressing an oleosin-protein A fusion protein, said oleosin-protein A fusion protein being bound to an immunoglobulin. This Figure corresponds to Figure 15 of SemBioSys Genetics Inc.'s U.S. patent No. 6,924,363.

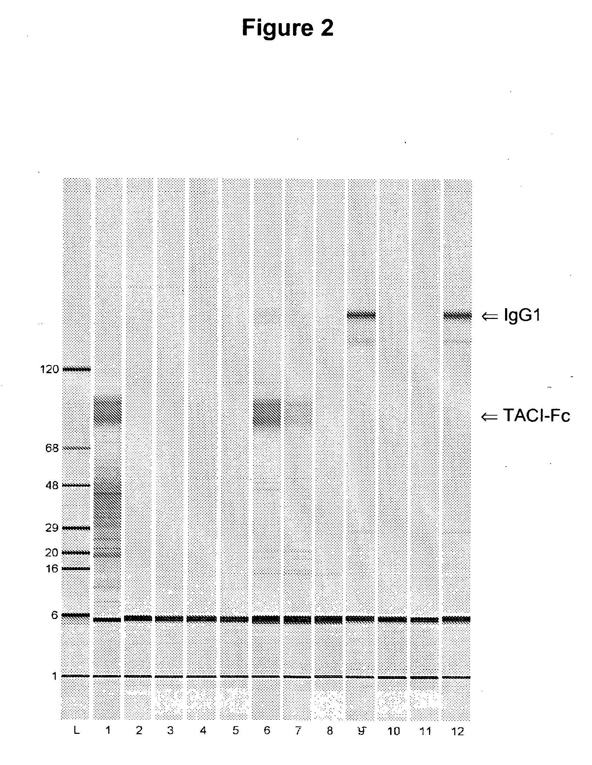

Figure 2 shows the microfluidic electrophoresis results of the capture of TACI-Fc5 harvests (lanes 1 to 8) and Human IgG1 kappa samples (lanes 9 to 12) obtained when performing the experiment described in Example 1. Lane L corresponds to the ladder. Lane 1 corresponds to the TACI-Fc-containing sample before purification. Lanes 2 and 10 corresponds to the "flow-through", i.e., the fraction of the TACI-Fc5 and of the Human IgG1 kappa samples that did not bind to the oilbodies. Lanes 3 to 5 correspond to the harvests obtained after the three consecutive washes described in Example 1.3. Lanes 6 to 8 correspond to the harvests obtained after the three consecutive elutions described in Example 1.4. Lane 9 corresponds to the IgG1-containing sample before purification. Lane 11 corresponds to the fraction obtained after the wash step. Lane 12 corresponds to the fraction obtained after the first elution step.
Figure 3 shows the loading capacity analysis of TACI-Fc harvest on StratoCapture^{™} oilbodies (see Example 2).

### BRIEF DESCRIPTION OF THE SEQUENCE LISTING

SEQ ID NO: 1 is the full length sequence of the human TACI receptor (e.g. described in WO 98/39361).
SEQ ID NO: 2 is a preferred TACI-Fc protein according to the invention, comprising sequences derived from the extracellular portion of TACI and a human IgG₁ Fc portion (e.g. described in WO 02/094852).
SEQ ID NO: 3 is a polynucleotide encoding the TACI-Fc protein of SEQ ID NO: 2.
SEQ ID NO: 4 is a preferred oleosin-protein A fusion protein according to the invention (e.g. described in U.S. patent No. 6,924,363).

### DETAILED DESCRIPTION OF THE INVENTION

The present invention is based on the development of a purification method for TACI-Fc. TACI-Fc binds and elutes from oilbodies using the experimental conditions developed in the frame of the present invention (see Example 1). The best results were obtained when:
- 5 grams of TACI-Fc were loaded per gram of dry-weight oilbodies (see example 2); and
- the oilbodies were eluted at a pH of about 3.5 (see Example 3).

In addition, oilbodies surprisingly and advantageously demonstrated a higher selectivity for TACI-Fc dimers (96% in post-capture fraction) than protein A chromatography (see Example 3).

The oilbody technology offers the potential to transform the capital intensive, expensive batch chromatographic purification process of a Fc-fusion protein such as, e.g., TACI-Fc, into a convenient low-cost process based on protein A oilbodies as disposable reagent.

Therefore, a first aspect of the invention relates to a method for purifying a TACI-Fc protein comprising the following steps:
a) mixing a solution comprising protein A oilbodies with a sample comprising said TACI-Fc protein in such a way as to obtain a final ratio of mg of TACI-Fc protein per mg of dry-weight oilbodies within a range of about 1 to about 6; and
b) separating said protein A oilbodies from said TACI-Fc protein by adding an aqueous solution having a pH comprised within a range of 3.5 to 3.9,
wherein the solution comprising TACI-Fc obtained at the end of step (b) comprises more than 93%, 94%, 95% or 96% of TACI-Fc dimers.

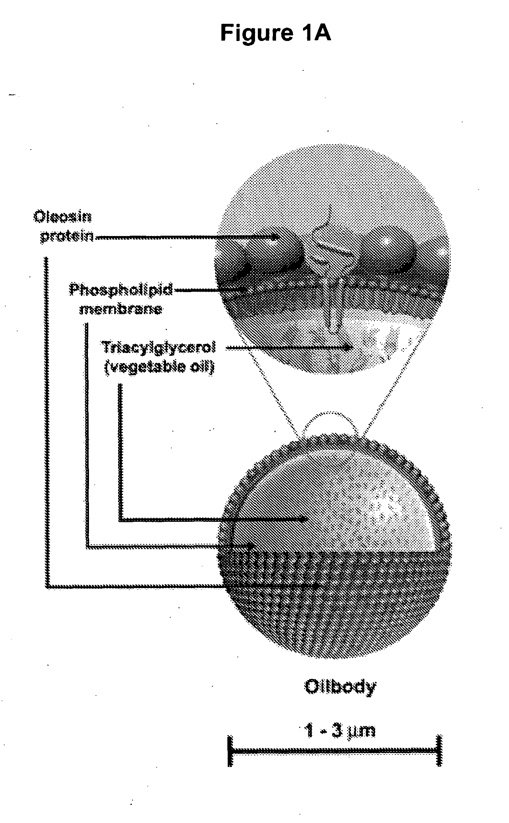

Oilbodies are protein-coated lipospheres that naturally form in plant seeds to function in triglyceride (oil) storage (see Figure 1A). Oilbodies are small, spherical, subcellular organelles encapsulating stored triacylglycerides, an energy reserve used by many plants. Although they are found in most plants and in different tissues, they are particularly abundant in the seeds of oilseeds where they range in size from under one micron to a few microns in diameter. Oilbodies are comprised of the triacylglycerides surrounded by a half-unit membrane of phospholipids and embedded with a unique type of protein known as an oilbody protein. The term "oilbody" or "oilbodies" as used herein includes any or all of the triacylglyceride, phospholipid or protein components present in the complete structure. The term "oilbody protein" as used herein means a protein that is naturally present in an oilbody. In plants, the predominant oilbody proteins are termed "oleosins". Oleosins have been cloned and sequenced from many plant sources including corn, rapeseed, carrot and cotton. The oleosin protein appears to be comprised of three domains; the two ends of the protein, N- and C-termini, are largely hydrophilic and reside on the surface of the oilbody exposed to the cytosol while the highly hydrophobic central core of the oleosin is firmly anchored within the membrane and triacylglyceride. Oleosins from different species represent a small family of proteins showing considerable amino acid sequence conservation, particularly in the central region of protein. Within an individual species, a small number of different isoforms may exist. Oilbodies, oilbody-associated proteins and/or oleosins may be genetically engineered in order to recombinant proteins be expressed on the surface of the oilbodies.

As used herein, the term "protein A oilbody" refers to an oilbody that has been genetically engineered so that Protein A or Protein A domains that bind to IgG are expressed on the surface of the genetically modified oilbodies. Such protein A oilbodies are described e.g. in U.S. patent No. 6,924,363 (Moloney et al., 2005) and are for example provided by SemBioSys Genetics Inc. under the trade name of StratoCapture^{™}

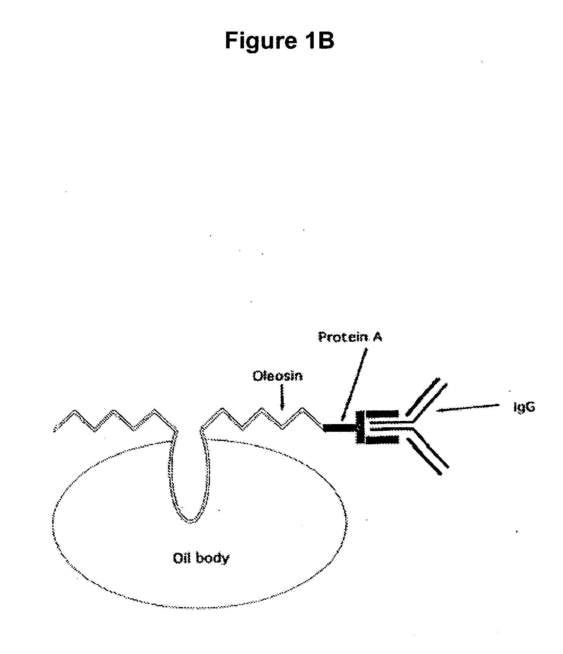

In a preferred embodiment according to the invention, the Protein A or Protein A domains that bind to IgG are fused to an oleosin (see Figure 1B). Such fusion proteins are referred to as "oleosin-protein A fusion proteins" throughout the present specification. The protein A oilbody of this preferred embodiment is referred to as an "oleosin-protein A oilbody". The protein of SEQ ID NO: 4 is a preferred oleosin-protein A fusion protein in accordance with the invention, which consists of a protein A domain which has been fused to the 18 kDa Arabidopsis oleosin (van Rooijen et al., 1992). This oleosin-protein A fusion protein may be constructed as described e.g. in Example 9 of U.S. patent No. 6,924,363. A synthetic protein A sequence encoding a protein capable of binding to IgG was synthesized based on reported sequence information (pRIT2T, protein A gene fusion vector; Pharmacia). The resulting protein A fragment was ligated into a pUC19 plasmid carrying the Arabidopsis oleosin gene comprised of an 867 bp upstream promoter region followed by the coding region (with its associated intron) from which the translational stop codon had been removed. The 3' end of the construct contains the nopaline synthase transcriptional terminator. A spacer sequence encoding a recognition sequence for the endoprotease thrombin was incorporated immediately downstream of the oleosin coding sequence. The protein A gene sequence was introduced between this spacer sequence and the terminator sequence. In the final expression construct the oleosin and protein A coding regions were fused in the same reading frame, leading to a sequence coding for the protein of SEQ ID NO: 4. The entire construct was then excised from the pUC19 plasmid and subcloned into the plant transformation vector pCGN1559 (McBride and Summerfelt, 1990) carrying a neomycin phosphotransferase gene under the control of the 35S CaMV promoter. The resulting plasmid was introduced in Agrobacterium (strain EHA101).

The protein A oilbodies of the invention are preferably obtained from a seed plant and more preferably from the group of plant species comprising: thale cress (Arabidopsis thaliana), rapeseed (Brassica spp.), soybean (Glycine max), sunflower (Helianthus annuus), oil palm (Elaeis guineeis), cottonseed (Gossypium spp.), goundnut (Arachis hypogaca), coconut (Cocus nucifera), castor (Ricinus communis), safflower (Carthamus linctorius), mustard (Brassica spp. and Sinapis alba), coriander (Coriandrum sativum) linseed/flax (Linum usitatissimum), and maize (Zea mays). Plants are grown and allowed to set seed using agricultural cultivation practises well known to a person skilled in the art. After harvesting the seed and removal of foreign material such as stones or seed hulls, for by example sieving, seeds are preferably dried and subsequently processed by mechanical pressing, grinding or crushing. The protein A oilbody fraction may be obtained from the crushed seed fraction by capitalization on separation techniques which exploit differences in density between the protein A oilbody fraction and the aqueous fraction, such as centrifugation, or using size exclusion-based separation techniques, such as membrane filtration, or a combination of both of these. Typically, seeds are thoroughly ground in five volumes of a cold aqueous buffer. A wide variety of buffer compositions may be employed, provided that they do not contain high concentrations of strong organic solvents such as acetone or diethyl ether, as these solvents may disrupt the protein A oilbodies. The solution density of the grinding buffer may be increased with the addition of 0.4-0.6 M sucrose, in order to facilitate washing as described below. The grinding buffer will also typically contain 0.5 M NaCl to help remove soluble proteins that are not integrally bound to the protein A oilbody surface. Following grinding, the homogenate is centrifuged resulting in a pellet of particulate and insoluble matter, an aqueous phase containing soluble components of the seed, and a surface layer comprised of protein A oilbodies with their associated proteins. The protein A oilbody layer is skimmed from the surface and thoroughly resuspended in one volume of fresh grinding buffer. It is important that aggregates of protein A oilbodies are dissociated as thoroughly as possible in order to ensure efficient removal of contaminants in the subsequent washing steps. The resuspended protein A oilbody preparation is layered under a flotation solution of lower density (e.g. water, aqueous buffer) and centrifuged, again, separating protein A oilbody and aqueous phases. The washing procedure is typically repeated at least three times, after which the protein A oilbodies are deemed to be sufficiently free of contaminating soluble proteins as determined by gel electrophoresis. It is not necessary to remove all of the aqueous phase and to the final preparation water or 50 mM Tris-HCl pH 7.5 may be added and if so desired the pH may be lowered to pH 2 or raised to pH 10. Protocols for isolating oilbodies from oil seeds are available in the art (Moloney et al., 2005). Protein A oilbodies other than those derived from plants may also be used in the present invention. A system functionally equivalent to plant genetically modified oilbodies and oleosins has been described in bacteria (Pieper-Furst et al., 1994), algae, and fungi (Ting et al., 1997). Protein A oilbodies from these organisms, as well as those that may be discovered in other living cells by a person skilled in the art, may also be employed according to the subject invention.

As used herein, the terms "TACI-Fc" and "a TACI-Fc protein" refer to a fusion protein comprising an extracellular domain of TACI, or a fragment thereof binding at least one of Blys or APRIL, fused to an Fc region of an immunoglobulin. The Fc region of an immunoglobulin is also referred to as the "Fc-moiety" of TACI-Fc. An assay for testing the capability of binding to Blys or APRIL is described e.g. in (Hymowitz et al., 2005). TACI is preferably human TACI. SEQ ID NO: 1 corresponds to the amino acid sequence of human full-length TACI receptor (also SwissProt Accession No. 014836). Preferably, the TACI-derived fragment comprises at least amino acids 33 to 67 of SEQ ID NO: 2 and/or amino acids 70 to 104 of SEQ ID NO: 1. In a preferred embodiment, the TACI-derived fragment comprises or consist of amino acids 1 to 166 of SEQ ID NO: 1 or amino acids 30 to 166 of SEQ ID NO: 1, or amino acids 30 to 119 of SEQ ID NO: 1, or amino acids 30 to 110 of SEQ ID NO: 1. All of those TACI-derived fragment are preferred for the preparation of the TACI protein to be purified by the method of the invention and are combined with an Fc-moiety. A highly preferred TACI protein to be purified in accordance with the present invention comprises or consists of SEQ ID NO: 2.

Hence, it is highly preferred that TACI-Fc comprises a polypeptide selected from:
a) amino acids 34 to 66 of SEQ ID NO: 1;
b) amino acids 71 to 104 of SEQ ID NO: 1;
c) amino acids 34 to 104 of SEQ ID NO: 1;
d) amino acids 30 to 110 of SEQ ID NO: 1;
e) SEQ ID NO: 2;
f) a polypeptide encoded by a polynucleotide hybridizing to the complement of SEQ ID NO: 3 under highly stringent conditions;
g) a mutein of any of (a), (b), (c), (d), (e), or (f) having at least 80 % or 85 % or 90 % or 95 % or 99% sequence identity to the polypeptide of (c), (d), (e) or (f); and
h) a functional derivative of any of (a), (b), (c), (d), (e), (f) or (g).
wherein the polypeptide binds to at least one of Blys or APRIL.

The term "muteins", as used herein, refers to analogs of TACI-Fc, in which one or more of the amino acid residues of TACI-Fc are replaced by different amino acid residues, or are deleted, or one or more amino acid residues are added to the original sequence of TACI-Fc without changing considerably the activity of the resulting products as compared with the original TACI-Fc. These muteins are prepared by known synthesis and/or by site-directed mutagenesis techniques, or any other known technique suitable therefore.

Muteins in accordance with the present invention include proteins encoded by a nucleic acid, such as DNA or RNA, which hybridizes to DNA or RNA, which encodes a TACI-Fc protein according to SEQ ID NO: 2 under stringent conditions. An example for a DNA sequence encoding a TACI-Fc is SEQ ID NO: 3.

The term "stringent conditions" refers to hybridization and subsequent washing conditions, which those of ordinary skill in the art conventionally refer to as "stringent". See Ausubel et al., Current Protocols in Molecular Biology, supra, Interscience, N.Y., §§6.3 and 6.4 (1987, 1992). Without limitation, examples of stringent conditions include washing conditions 12-20°C below the calculated Tm of the hybrid under study in, e.g., 2 x SSC and 0.5% SDS for 5 minutes, 2 x SSC and 0.1% SDS for 15 minutes; 0.1 x SSC and 0.5% SDS at 37°C for 30-60 minutes and then, a 0.1 x SSC and 0.5% SDS at 68°C for 30-60 minutes. Those of ordinary skill in this art understand that stringency conditions also depend on the length of the DNA sequences, oligonucleotide probes (such as 10-40 bases) or mixed oligonucleotide probes. If mixed probes are used, it is preferable to use tetramethyl ammonium chloride (TMAC) instead of SSC. See Ausubel, supra.

Identity reflects a relationship between two or more polypeptide sequences or two or more polynucleotide sequences, determined by comparing the sequences. In general, identity refers to an exact nucleotide to nucleotide or amino acid to amino acid correspondence of the two polynucleotides or two polypeptide sequences, respectively, over the length of the sequences being compared. For sequences where there is not an exact correspondence, a "% identity" may be determined. In general, the two sequences to be compared are aligned to give a maximum correlation between the sequences. This may include inserting "gaps" in either one or both sequences, to enhance the degree of alignment. A % identity may be determined over the whole length of each of the sequences being compared (so-called global alignment), that is particularly suitable for sequences of the same or very similar length, or over shorter, defined lengths (so-called local alignment), that is more suitable for sequences of unequal length. Methods for comparing the identity and homology of two or more sequences are well known in the art. Thus for instance, programs available in the Wisconsin Sequence Analysis Package, version 9.1(Devereux et al., 1984), for example the programs BESTFIT and GAP, may be used to determine the % identity between two polynucleotides and the % identity and the % homology between two polypeptide sequences. BESTFIT uses the "local homology" algorithm of (Smith and Waterman, 1981) and finds the best single region of similarity between two sequences. Other programs for determining identity and/or similarity between sequences are also known in the art; for instance the BLAST family of programs (Altschul et al., 1990; Altschul et al., 1997), accessible through the home page of the NCBI at world wide web site ncbi.nlm.nih.gov) and FASTA (Pearson, 1990). It is highly preferred that the % identity between two sequences is determined using the KERR algorithm (Dufresne et al., 2002), for example by using a bioinformatic tool such as e.g. GenePAST.

Any such mutein preferably has a sequence of amino acids sufficiently duplicative of that of TACI-Fc, such as to have substantially similar ligand binding activity as a protein of SEQ ID NO: 2. For instance, one activity of TACI is its capability of binding to Blys or APRIL (Hymowitz et al., 2005). As long as the mutein has substantial APRIL or Blys binding activity, it can be considered to have substantially similar activity to TACI-Fc. Thus, it can be easily determined by the person skilled in the art whether any given mutein has substantially the same activity as a protein of SEQ ID NO: 2 by means of routine experimentation.

In a preferred embodiment, any such mutein has at least 50 %, at least 60 %, at least 70 %, at least 75 %, at least 80%, at least 85 %, at least 90%, at least 95 %, or at least 99 % identity or homology thereto.

Preferred changes for muteins in accordance with the present invention are what are known as "conservative" substitutions. Conservative amino acid substitutions of TACI-Fc may include synonymous amino acids within a group which have sufficiently similar physicochemical properties that substitution between members of the group will preserve the biological function of the molecule (Grantham, 1974). It is clear that insertions and deletions of amino acids may also be made in the above-defined sequences without altering their function, particularly if the insertions or deletions only involve a few amino acids, e.g., under thirty, under twenty, or preferably under ten, and do not remove or displace amino acids which are critical to a functional conformation, e.g., cysteine residues. Proteins and muteins produced by such deletions and/or insertions come within the purview of the present invention.

Preferably, the conservative amino acid groups are those defined in Table 2. More preferably, the synonymous amino acid groups are those defined in Table 3; and most preferably the synonymous amino acid groups are those defined in Table 4.

**TABLE 2**

| Preferred Groups of Synonymous Amino Acids | |
|---|---|
| **Amino Acid** | **Synonymous Group** |
| Ser | Ser, Thr, Gly, Asn |
| Arg | Arg, Gin, Lys, Glu, His |
| Leu | Ile, Phe, Tyr, Met, Val, Leu |
| Pro | Gly, Ala, Thr, Pro |
| Thr | Pro, Ser, Ala, Gly, His, Gln, Thr |
| Ala | Gly, Thr, Pro, Ala |
| Val | Met, Tyr, Phe, Ile, Leu, Val |
| Gly | Ala, Thr, Pro, Ser, Gly |
| Ile | Met, Tyr, Phe, Val, Leu, Ile |
| Phe | Trp, Met, Tyr, Ile, Val, Leu, Phe |
| Tyr | Trp, Met, Phe, Ile, Val, Leu, Tyr |
| Cys | Ser, Thr, Cys |
| His | Glu, Lys, Gln, Thr, Arg, His |
| Gin | Glu, Lys, Asn, His, Thr, Arg, Gln |
| Asn | Gin, Asp, Ser, Asn |
| Lys | Glu, Gin, His, Arg, Lys |
| Asp | Glu, Asn, Asp |
| Glu | Asp, Lys, Asn, Gln, His, Arg, Glu |
| Met | Phe, Ile, Val, Leu, Met |
| Trp | Trp |

**TABLE 3**

| More Preferred Groups of Synonymous Amino Acids | |
|---|---|
| **Amino Acid** | **Synonymous Group** |
| Ser | Ser |
| Arg | His, Lys, Arg |
| Leu | Leu, Ile, Phe, Met |
| Pro | Ala, Pro |
| Thr | Thr |
| Ala | Pro, Ala |
| Val | Val, Met, Ile |
| Gly | Gly |
| Ile | Ile, Met, Phe, Val, Leu |
| Phe | Met, Tyr, Ile, Leu, Phe |
| Tyr | Phe, Tyr |
| Cys | Cys, Ser |
| His | His, Gln, Arg |
| Gln | Glu, Gln, His |
| Asn | Asp, Asn |
| Lys | Lys, Arg |
| Asp | Asp, Asn |
| Glu | Glu, Gln |
| Met | Met, Phe, Ile, Val, Leu |
| Trp | Trp |

**TABLE 4**

| Most Preferred Groups of Synonymous Amino Acids | |
|---|---|
| **Amino Acid** | **Synonymous Group** |
| Ser | Ser |
| Arg | Arg |
| Leu | Leu, Ile, Met |
| Pro | Pro |
| Thr | Thr |
| Ala | Ala |
| Val | Val |
| Gly | Gly |
| Ile | Ile, Met, Leu |
| Phe | Phe |
| Tyr | Tyr |
| Cys | Cys, Ser |
| His | His |
| Gln | Gln |
| Asn | Asn |
| Lys | Lys |
| Asp | Asp |
| Glu | Glu |
| Met | Met, Ile, Leu |
| Trp | Met |

"Functional derivatives" as used herein cover derivatives of TACI-Fc to be purified in accordance with the present invention, which may be prepared from the functional groups which occur as side chains on the residues or the N- or C-terminal groups, by means known in the art, and are included in the invention as long as they remain pharmaceutically acceptable, i.e. they do not destroy the activity of TACI-Fc which is substantially similar to the activity of the unmodified TACI-Fc protein as defined above, and do not confer toxic properties on compositions containing it.

Functional derivatives of TACI-Fc can e.g. be conjugated to polymers in order to improve the properties of the protein, such as the stability, half-life, bioavailability, tolerance by the human body, or immunogenicity. To achieve this goal, TACI-Fc may be linked e.g. to polyethylene glycol (PEG). PEGylation may be carried out by known methods, described in WO 92/13095, for example.

Functional derivatives may also, for example, include aliphatic esters of the carboxyl groups, amides of the carboxyl groups by reaction with ammonia or with primary or secondary amines, N-acyl derivatives of free amino groups of the amino acid residues formed with acyl moieties (e.g. alkanoyl or carbocyclic aroyl groups) or O-acyl derivatives of free hydroxyl groups (for example that of seryl or threonyl residues) formed with acyl moieties.

The Fc-moiety of TACI-Fc may be derived from a human or animal immunoglobulin (Ig) that is preferably an IgG. The IgG may be an IgG₁, IgG₂, IgG₃ or IgG₄. It is also preferred that the Fc-moiety is derived from the heavy chain of an immunoglobulin, preferably an IgG. More preferably, the Fc-moiety comprises a portion, such as e.g. a domain, of an immunoglobulin heavy chain constant region. Such Ig constant region preferably comprises at least one Ig constant domain selected from any of the hinge, CH2, CH3 domain, or any combination thereof. It is preferred that the Fc-moiety comprises at least a CH2 and CH3 domain. It is further preferred that the Fc-moiety comprises the IgG hinge region, the CH2 and the CH3 domain. Preferably, TACI-Fc comprises a heavy chain constant region of an immunoglobulin, more preferably a human constant region. Most preferably, the immunoglobulin is an IgG₁. It, is also preferred that the constant region comprises a hinge, a CH2 domain and a CH3 domain.

A preferred embodiment is directed to a method of purifying TACI-Fc according to the invention wherein the solution comprising TACI-Fc obtained at the end of step (b) comprises more than 97%, 98% or 99% of dimeric forms of TACI-Fc. Indeed, as shown in Table 6 of Example 3, the method of the invention demonstrated a higher selectivity for TACI-Fc dimers than protein A chromatography.

In a preferred embodiment of the present purification process, one or more wash steps are performed. As used herein, the term "wash" refers to a method comprising the steps of:
(i) suspending the protein A oilbodies in an aqueous solution;
(ii) centrifuging the suspension; and
(iii) removing the underlying aqueous phase, the upper phase comprising the protein A oilbodies.
Such a wash step may for example be performed between step (a) and (b) of the method in accordance with the invention, and/or prior to performing step (a).

Centrifugations may be carried out at, e.g., 5 000 x g, 6 000 x g, 7 000 x g, 8 000 x g, 9 000 x g, 10 000 x g, 11 000 x g, 12 000 x g, 13 000 x g, 14 000 x g, 15 000 x g, 16 000 x g, 17 000 x g, 18 000 x g, 19 000 x g or 20 000 x g for about 5, 6, 7, 8, 9, 10, 11, 12, 13, 14, 15, 16, 17, 18, 19 or 20 mn. Preferably, centrifugations are carried out at 10 000 x g for 10 mn.

Preferably, the protein A oilbodies are washed with a buffer at a pH within a range of about 5 to about 9, of about 5.5 to about 8.5, of about 6 to about 8, of about 6.5 to about 7.5. Most preferably, the protein A oilbodies are washed with a buffer at a pH of about 7.

Also preferably, the buffer used for washing the protein A oilbodies is a phosphate buffer.

Also preferably, the wash step is repeated at least 2, 3, 4 or 5 times, most preferably 3 times.

In a further preferred embodiment of the present purification process, the final ratio of mg of TACI-Fc protein per mg of dry-weight protein A oilbodies in step (a) is within a range of about 1 to about 6, of about 1.5 to about 5.5, of about 2 to about 5, of about 2.5 to about 4.8, of about 3 to about 4.6, of about 3.5 to about 4.5, or of about 4 to 5.5. Preferably, the final ratio of mg of TACI-Fc protein per mg of dry-weight protein A oilbodies in step (a) is of about 4, of about 4.25, of about 4.5, of about 4.75, of about 5, of about 5.25, of about 5.5, of about 5.75 or of about 6. When purifying TACI-Fc, it is highly preferred that the final ratio of mg of TACI-Fc per mg of dry-weight protein A oilbodies in step (a) is of about 4, of about 5 or of about 5.5.

Step (b) may be referred to as "elution step", and the separation of the protein A oilbodies from the TACI-Fc protein may for example be performed as follows:
(i) suspending the protein A oilbodies in an aqueous solution;
(ii) centrifuging the suspension; and
(iii) collecting the underlying aqueous phase comprising the purified TACI-Fc protein, e.g. using a syringe.

In a preferred embodiment of the present purification process, step (b) is performed by adding an aqueous solution having a pH of about 3.5, 3.6, 3.7, 3.8, 3.8 or 3.9, most preferably about 3.5.

Preferably, the aqueous solutions used at step (b) comprises acetate, e.g. about 100, 125, 150, 175, 200, 225, 250, 275 or 300 mM acetate.

Also preferably, step (b) step is repeated at least 2, 3, 4 or 5 times, most preferably 3 times.

The purification process in accordance with the present invention may further comprise a step (c) of filtering the solution comprising said TACI-Fc protein obtained at the end of step (b). Preferably, the pore size of the filter is of 0.05, 0.1, 0.2, 0.3, 0.4 or 0.5 µm.

The purification process in accordance with the present invention may further comprise a step (d) of neutralizing the solution obtained at the end of step (c) (or of step (b) in case step (c) is not performed). The solution may be neutralized using e.g. NaOH. Preferably, NaOH 1 M is used.

Optionally, the purification process in accordance with the present invention may comprise one or more supplemental purification steps using any technique known in the prior art such as, e.g., a cation exchange chromatography, an anion exchange chromatography and/or a hydroxyapatite chromatography. These supplemental purification steps are commonly referred to as a "polishing steps" by the skilled in the art. Specifically, the methods known in the art for purifying Fc-fusion proteins comprise a first purification step by Protein A, Protein G or Protein L affinity chromatography and further polishing steps aiming at obtaining such purity as to be suitable for human administration. In accordance with the present invention, the purification step by Protein A, Protein G or Protein L affinity chromatography is replaced by a purification step using protein A oilbodies and the polishing steps are performed as already known in the art. For example, the polishing steps may comprise the step of eliminating free Fc-moieties by cation exchange chromatography as disclosed in EP application No. 06 119 611.9. For example, the polishing steps may be performed as disclosed in EP application No. 06 119 610.1. This would lead, e.g., to a TACI-Fc purification process comprising the following steps:
(i) purifying a fluid comprising TACI-Fc using protein A oilbodies as described in the fame of the present invention;
(ii) subjecting the eluate of step (a) to cation exchange chromatography;
(iii) subjecting the eluate of step (b) to anion exchange chromatography; and
(iv) subjecting the flow-through of step (c) to hydroxyapatite chromatography and collecting the eluate to obtain purified TACI-Fc.

Optionally, the TACI-Fc protein is formulated into a pharmaceutical composition at the end of the purification process in accordance with the present invention, i.e., one or more pharmaceutically acceptable carriers and/or excipients or the like are added to the solution comprising the TACI-Fc protein that is obtained at the end of the purification process in accordance with the present invention.

The definition of "pharmaceutically acceptable" is meant to encompass any carrier, which does not interfere with effectiveness of the biological activity of the active ingredient and that is not toxic to the host to which it is administered. For example, for parenteral administration, the active protein(s) may be formulated in a unit dosage form for injection in vehicles such as saline, dextrose solution, serum albumin and Ringer's solution.

The active ingredients of the pharmaceutical composition according to the invention can be administered to an individual in a variety of ways. The routes of administration include intradermal, transdermal (e.g. in slow release formulations), intramuscular, intraperitoneal, intravenous, subcutaneous, oral, intracranial, epidural, topical, rectal, and intranasal routes. Any other therapeutically efficacious route of administration can be used, for example absorption through epithelial or endothelial tissues or by gene therapy wherein a DNA molecule encoding the active agent is administered to the patient (e.g. via a vector), which causes the active agent to be expressed and secreted in vivo. In addition, the protein(s) according to the invention can be administered together with other components of biologically active agents such as pharmaceutically acceptable surfactants, excipients, carriers, diluents and vehicles.

For parenteral (e.g. intravenous, subcutaneous, intramuscular) administration, the active protein(s) can be formulated as a solution, suspension, emulsion or lyophilized powder in association with a pharmaceutically acceptable parenteral vehicle (e.g. water, saline, dextrose solution) and additives that maintain isotonicity (e.g. mannitol) or chemical stability (e.g. preservatives and buffers). The formulation is sterilized by commonly used techniques.

The therapeutically effective amounts of the active protein(s) will be a function of many variables, including the the affinity of the TACI-Fc protein for its ligand, the route of administration and the clinical condition of the patient. "therapeutically effective amount" is such that when administered, the TACI-Fc protein results in inhibition of the corresponding ligand. In the case of TACI-Fc the ligand may be, e.g., Blys and/or APRIL. The dosage administered, as single or multiple doses, to an individual will vary depending upon a variety of factors, including pharmacokinetic properties of the TACI-Fc protein, the route of administration, patient conditions and characteristics (sex, age, body weight, health, size), extent of symptoms, concurrent treatments, frequency of treatment and the effect desired. Adjustment and manipulation of established dosage ranges are well within the ability of those skilled in the art, as well as *in vitro* and *in vivo* methods of determining the inhibition of its ligand of the therapeutic moiety in an individual.

Purified TACI-Fc proteins may be used in an amount of about 0.001 to 100 mg/kg or about 0.01 to 10 mg/kg or body weight, or about 0. 1 to 5 mg/kg of body weight or about 1 to 3 mg/kg of body weight or about 2 mg/kg of body weight.

In further preferred embodiments, the purified TACI-Fc protein is administered daily or every other day or three times per week or once per week. The daily doses are usually given in divided doses or in sustained release form effective to obtain the desired results. Second or subsequent administrations can be performed at a dosage which is the same, less than or greater than the initial or previous dose administered to the individual. A second or subsequent administration can be administered during or prior to onset of the disease.

In a preferred embodiment TACI-Fc is atacicept. Purified atacicept may preferably be used for preparation of a medicament for treatment and/or prevention of a number of diseases or disorders. Such diseases or disorders are preferably selected from autoimmune disorders such as systemic lupus erythematosus (SLE), rheumatoid arthritis (RA), as well as for treatment of multiple sclerosis (MS). Purified TACI-Fc may also be used for treatment of cancer, such as hematological malignancies such as multiple myeloma (MM) and/or non-Hodgkin's lymphoma (NHL), chronic lymphocytic leukemia (CLL) and Waldenstrom's macroglobulemia (WM).

In accordance with the present invention, the recombinant TACI-Fc protein may be produced in eukaryotic expression systems, such as yeast, insect, or mammalian cells, resulting in glycosylated TACI-Fc proteins. It is most preferred to express the TACI-Fc protein in mammalian cells such as animal cell lines, or in human cell lines. Chinese hamster ovary cells (CHO) or the murine myeloma cell line NS0 are examples of cell lines that are particularly suitable for expression of the TACI-Fc protein to be purified. The TACI-Fc protein can also preferably be produced in human cell lines, such as e.g. the human fibrosarcoma HT1080 cell line, the human retinoblastoma cell line PERC6, or the human embryonic kidney cell line 293, or a permanent amniocyte cell line as described e.g. in EP 1 230 354. If the TACI-Fc protein to be purified is expressed by mammalian cells secreting it, the starting material of the purification process of the invention is cell culture supernatant, also called harvest or crude harvest. If the cells are cultured in a medium containing animal serum, the cell culture supernatant also contains serum proteins as impurities.

Preferably, the TACI-Fc protein expressing and secreting cells are cultured under serum-free conditions. The TACI-Fc protein may also be produced in a chemically defined medium. In this case, the starting material of the purification process of the invention is serum-free cell culture supernatant that mainly contains host cell proteins as impurities. If growth factors are added to the cell culture medium, such as insulin, for example, these proteins will be eliminated during the purification process as well.

A second aspect of the invention is directed to a method of manufacturing a pharmaceutical composition comprising a TACI-Fc protein comprising the steps of:
a) purifying said TACI-Fc-protein according to the purification process in accordance with the present invention; and
b) formulating said TACI-Fc protein into a pharmaceutical composition,

The use of protein A oilbodies for purifying TACI-Fc is described.

### EXAMPLES

### Example 1: Protocol for purification of TACI-Fc using oilbodies

### 1.1. Wash of oilbodies prior to use

StratoCapture^{™} oilbodies were obtained from SemBioSys Genetics Inc. The oilbodies were first evenly resuspended in the solution comprising them by gently rotating the tube. The oilbodies solution were centrifuged at 10 000 x g for 10 minutes and the underlying aqueous phase was removed from the protein A oilbody layer:

The oilbodies were resuspended in a fresh equilibration buffer comprising 25 mM phosphate buffer at pH 7.0 using gentle pipetting or mixing. The added volume of equilibration buffer was identical to the aqueous phase that had previously been removed. The oilbodies solution was then centrifuged at 10 000 x g for 10 minutes and the underlying aqueous phase was removed from the oilbody layer. This equilibration step was performed three times.

### 1.2. Loading of the oilbodies with TACI-Fc

The solution comprising oilbodies was added to a sample comprising unpurified TACI-Fc in such a way as to obtain a final ratio of TACI-Fc per mg of dry-weight oilbody of about 4 mg. A sample comprising purified Human IgG1 kappa was used as a positive control. The solution was well but gently mixed by inversion. The tubes were centrifugated at 10 000 x g for 10 minutes. The undernatant was removed and discarded from the oilbodies using a needle syringe to poke through the fat pad layer.

### 1.3. Wash step

The fat pad oilbodies were gently re-suspended in a volume of wash buffer corresponding to once or twice the volume of the fat pad oilbodies. The wash buffer comprised 25 mM phosphate buffer at pH 7.0. The tube was centrifuged at 10 000 x g for 10 minutes. The underlying aqueous phase was removed and discarded from the oilbodies using a needle syringe to poke through the fat pad layer. The aqueous phase was filtered at 0.2 µm and aliquoted. This wash step was performed three times.

### 1.4. Elution step

The fat pad oilbodies were re-suspended in an appropriate volume in a volume of elution buffer corresponding to once or twice the volume of the fat pad oilbodies. The elution buffer compried 200 mM acetate buffer at pH 3.5. The tube was centrifuged at 10 000 x g for 10 minutes and the undernatant collected using a syringe. The undernatant comprising the purified Taci-Fc was filtered at 0.2 µm and neutralized at pH 7.0 using NaOH 1M. This elution step was performed three times.

### 1.5. Results

Results were analysed by microfluidic electrophoresis (Caliper system) and are shown on Figure 2. These microfluidic electrophoresis results demonstrate that TACI-Fc can efficiently be purified using the above protocol.

### Example 2: Determination of the optimal loading capacity for Taci-Fc

The loading capacity of oilbodies is an important parameter to optimize. When loading too much TACI-Fc per mg dry weight (DW) of oilbodies (OB), the TACI-Fc proteins will not bind to the oilbodies and be lost in the "flow-through", i.e., the unbound fraction of the sample comprising TACI-Fc. The % of unbound TACI-Fc that is lost in the flow-through is referred to as the "% breakthrough". To the contrary, when loading few TACI-Fc per mg DW OB, a high percentage of oilbodies does not bind to any TACI protein and this leads to a cost-ineffective process. Therefore, the optimal loading capacity of oilbodies was determined for TACI-Fc.

The loading capacity of oilbodies was determined by loading increasing amounts of TACI-Fc on oilbodies. The process conditions are described in Example 1 except for the value of the final ratio of mg of TACI-Fc per mg of dry-weight oilbody in paragraph 1.2. The loading capacity was determined by measuring the level of TACI-Fc in the unbound fraction. The capacity was expressed in mg of TACI-Fc per mg DW OB.

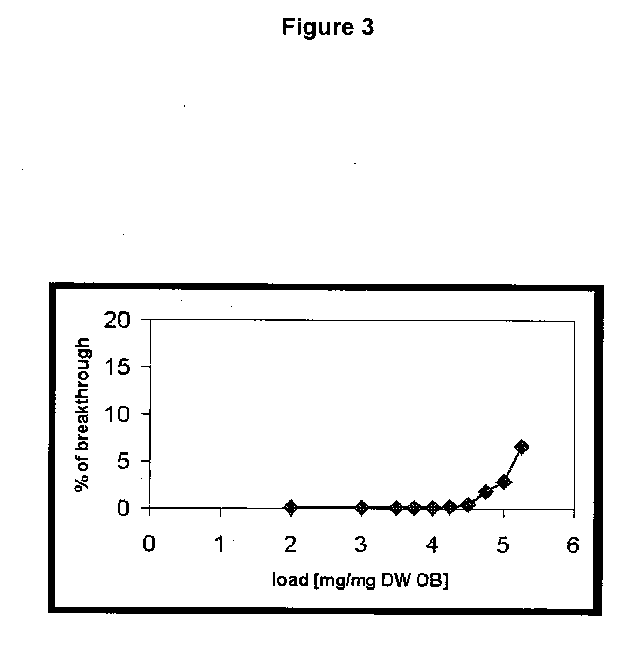

The results are shown on Figure 3. This experiment allows concluding that:
- Up til about 4 mg of TACI-Fc per mg DW OB may be loaded when working at 0% breakthrough;
- Up til about 5 mg of TACI-Fc per mg DW OB may be loaded when working at 5% breakthrough; and
- Up til about 5.5 mg of TACI-Fc per mg DW OB may be loaded when working at 10% breakthrough.

### Example 3: Optimization of the elution step

The performance of the elution step was explored by varying the pH of the elution buffer from 3.9 to 2.5. Equilibration, load and wash conditions were identical to those described in Example 1. Results are presented in Table 5.

**Table 5: Impact of the pH at elution on the purification of TACI-Fc**

| **pH at elution** | **Yield [%]** | **% dimer at elution** | **HCP [ppm]** |
|---|---|---|---|
| 3.9 | 41 | 95 | 15'000 |
| 3.9 | 53 | 96 | 13'000 |
| 3.0 | 54 | 92 | 30'000 |
| 2.5 | Oilbodies were solubilized and no phase separation was obtained. | | |

As illustrated on Table 5, decreasing the pH does not impact the yield at elution but decreases the clearance of HCP.

Table 6 illustrates a comparison of the performances of oilbodies with protein A chromatography.

**Table 6: Comparison of oilbodies and protein A chromatography**

| **Technology** | **Yield [%]** | **% dimer at load** | **% dimer at elution** | **HCP [ppm]** |
|---|---|---|---|---|
| StratoCapture | 47 | 76.5 | 96 | 14'000 |
| Protein A chromatography | 70 | 80.6 | 93 | 2000 |

In conclusion, StratoCapture material showed unique behaviour in terms of elution compared to protein A chromatography. On one hand, the clearance factor and the yield are lower than when using protein A chromatography processes. On the other hand, oilbodies demonstrated a higher selectivity for TACI-Fc dimers (96% in post-capture fraction) than protein A chromatography.

### Example 4: Analytical methods

TACI-Fc concentration in the clarified harvest was measured using a Biacore apparatus.

The level of aggregates in the clarified harvest was measured using Protein A Size-Exclusion Chromatography (PA-SEC).

TACI-Fc concentration in post-elution fractions and level of aggregates were measured by size-exclusion High Performance Liquid Chromatography (SE-HPLC).

Host Cell Proteins (HCP) level was measured by ELISA.

Microfluidic electrophoresis was performed using a Caliper LabChip®90 system.

The dry weight of oilbodies was measured as follows. The weigh boat was first measured. Approximately 0.3 to 0.5 ml of an oilbody sample was placed in the weigh boat The combined mass of boat and the wet sample was measured and recorded. The weigh boat containing the sample was placed in a drying oven set at 80 ± 5°C. After 4 hours to 4 days, the sample was removed from the sample from oven, and the combined mass of the weigh boat and dried sample was measured. The sample boat was placed back to the oven, dried for another half hour, and reweighted. If the weight of the sample had not varied, the measure corresponds to the dry weight of oilbodies. If the weight had changed, the sample was placed in the oven for another 30 minutes and reweighted. This was repeated until the sample weight did not change.

### Overall results advantages of the purification process of the present invention

TACI-Fc binds and elutes from oilbodies using the experimental conditions developed in the frame of the present invention (see Example 1). The best results were obtained when:
- 5 grams of TACI-Fc were loaded per gram of dry-weight oilbodies (see example 2); and
- the oilbodies were eluted at a pH of about 3.5 (see Example 3).

In addition, oilbodies surprisingly and advantageously demonstrated a higher selectivity for TACI-Fc dimers (96% in post-capture fraction) than protein A chromatography (see Example 3).

The use of the oilbody technology for purifying TACI-Fc is advantageous over a classical Protein A chromatography for several reasons.

Firstly, as mentioned above, protein A oilbodies demonstrated a higher selectivity for TACI-Fc dimers than classical Protein A chromatography. This is highly advantageous when TACI-Fc is intended for human administration since (i) only TACI-Fc dimers are pharmaceutically active; and (ii) TACI-Fc aggregates exhibit immunogenic properties.

Secondly, for purifying a given amount of TACI-Fc protein, the oilbody technology is much cheaper than the Protein A chromatography technology. Indeed, using cost models, it was estimated that using a purification process involving protein A oilbodies is advanatgeous. According to a cost model, the cost of the Protein A chromatography was estimated at 13 USD per gram of TACI-Fc. In contrast to this, the cost of the protein A oilbodies purification was estimated at 6.75 USD per gram of TACI-Fc.

Thirdly, the oilbody technology is more practical to put in place for large volumes of TACI-Fc proteins to be purified than a classical chromatography. Indeed, oilbodies are not soluble in water and the capture of antibody proteins using StratoCapture is therefore a simple liquid/liquid extraction, which makes the process convenient.

In summary, the oilbody technology offers the potential to transform the capital intensive, expensive batch chromatographic purification process of antibody into a convenient low-cost process based on protein A oilbodies as disposable reagent.

### REFERENCES

1. Akerstrom,B. and Bjorck,L. (1989). Protein L: an immunoglobulin light chain-binding bacterial protein. Characterization of binding and physicochemical properties. J. Biol. Chem. 264, 19740-19746.
2. Altschul,S.F., Gish,W., Miller,W., Myers,E.W., and Lipman,D.J. (1990). Basic local alignment search tool. J. Mol. Biol. 215, 403-410.
3. Altschul,S.F., Madden,T.L., Schaffer,A.A., Zhang,J., Zhang,Z., Miller,W., and Lipman,D.J. (1997). Gapped BLAST and PSI-BLAST: a new generation of protein database search programs. Nucleic Acids Res. 25, 3389-3402.
4. Armour,K.L., Clark,M.R., Hadley,A.G., and Williamson,L.M. (1999). Recombinant human IgG molecules lacking Fcgamma receptor I binding and monocyte triggering activities. Eur. J. Immunol. 29, 2613-2624.
5. Devereux,J., Haeberli,P., and Smithies,O. (1984). A comprehensive set of sequence analysis programs for the VAX. Nucleic Acids Res. 12, 387-395.
6. Dufresne,G., Takacs,L., Heus,H.C., Codani,J.J., and Duval,M. (2002). Patent searches for genetic sequences: how to retrieve relevant records from patented sequence databases. Nat. Biotechnol. 20, 1269-1271.
7. Grantham,R. (1974). Amino acid difference formula to help explain protein evolution. Science 185, 862-864.
8. Gross,J.A., Johnston,J., Mudri,S., Enselman,R., Dillon,S.R., Madden,K., Xu,W., Parrish-Novak,J., Foster,D., Lofton-Day,C., Moore,M., Littau,A., Grossman,A., Haugen,H., Foley,K., Blumberg,H., Harrison,K., Kindsvogel,W., and Clegg,C.H. (2000). TACI and BCMA are receptors for a TNF homologue implicated in B-cell autoimmune disease. Nature 404, 995-999.
9. Hahne,M., Kataoka,T., Schroter,M., Hofmann,K., Irmler,M., Bodmer,J.L., Schneider,P., Bornand,T., Holler,N., French,L.E., Sordat,B., Rimoldi,D., and Tschopp,J. (1998). APRIL, a new ligand of the tumor necrosis factor family, stimulates tumor cell growth. J. Exp. Med. 188, 1185-1190.
10. Hinton,P.R., Johlfs,M.G., Xiong,J.M., Hanestad,K., Ong,K.C., Bullock,C., Keller,S., Tang,M.T., Tso,J.Y., Vasquez,M., and Tsurushita,N. (2004). Engineered human IgG antibodies with longer serum half-lives in primates. J. Biol. Chem. 279, 6213-6216.
11. Hymowitz,S.G., Patel,D.R., Wallweber,H.J., Runyon,S., Yan,M., Yin,J., Shriver,S.K., Gordon,N.C., Pan,B., Skelton,N.J., Kelley,R.F., and Starovasnik,M.A. (2005). Structures of APRIL-receptor complexes: like BCMA, TACI employs only a single cysteine-rich domain for high affinity ligand binding. J. Biol. Chem. 280, 7218-7227.
12. Idusogie,E.E., Presta,L.G., Gazzano-Santoro,H., Totpal,K., Wong,P.Y., Ultsch,M., Meng,Y.G., and Mulkerrin,M.G. (2000). Mapping of the C1q binding site on rituxan, a chimeric antibody with a human IgG1 Fc. J. Immunol. 164, 4178-4184.
13. Idusogie,E.E., Wong,P.Y., Presta,L.G., Gazzano-Santoro,H., Totpal, K., Ultsch,M., and Mulkerrin,M.G. (2001). Engineered antibodies with increased activity to recruit complement. J. Immunol. 166, 2571-2575.
14. McBride,K.E. and Summerfelt,K.R. (1990). Improved binary vectors for Agrobacterium-mediated plant transformation. Plant Mol. Biol. 14, 269-276.
15. Moloney, Boothe, and van Rooijen. Oil bodies and associated proteins as affinity matrices. SemBioSys Genetics Inc. [U.S. patent No. 6,924,363]. 2-8-2005.
16. Moore,P.A., Belvedere,O., Orr,A., Pieri,K., LaFleur,D.W., Feng,P., Soppet,D., Charters,M., Gentz,R., Parmelee,D., Li,Y., Galperina,O., Giri,J., Roschke,V., Nardelli,B., Carrell,J., Sosnovtseva,S., Greenfield,W., Ruben,S.M., Olsen,H.S., Fikes,J., and Hilbert,D.M. (1999). BLyS: member of the tumor necrosis factor family and B lymphocyte stimulator. Science 285, 260-263.
17. Novak,A.J., Darce,J.R., Arendt,B.K., Harder,B., Henderson,K., Kindsvogel,W., Gross,J.A., Greipp,P.R., and Jelinek,D.F. (2004). Expression of BCMA, TACI, and BAFF-R in multiple myeloma: a mechanism for growth and survival. Blood 103, 689-694.
18. Pearson,W.R. (1990). Rapid and sensitive sequence comparison with FASTP and FASTA. Methods Enzymol. 183, 63-98.
19. Pieper-Furst,U., Madkour,M.H., Mayer,F., and Steinbuchel,A. (1994). Purification and characterization of a 14-kilodalton protein that is bound to the surface of polyhydroxyalkanoic acid granules in Rhodococcus ruber. J. Bacteriol. 176, 4328-4337.
20. Shields,R.L., Namenuk,A.K., Hong,K., Meng,Y.G., Rae,J., Briggs,J., Xie,D., Lai,J., Stadlen,A., Li,B., Fox,J.A., and Presta,L.G. (2001). High resolution mapping of the binding site on human IgG1 for Fc gamma RI, Fc gamma RII, Fc gamma RIII, and FcRn and design of IgG1 variants with improved binding to the Fc gamma R. J. Biol. Chem. 276, 6591-6604.
21. Smith,T.F. and Waterman,M.S. (1981). Identification of common molecular subsequences. J. Mol. Biol. 147, 195-197.
22. Steurer,W., Nickerson,P.W., Steele,A.W., Steiger,J., Zheng,X.X., and Strom,T.B. (1995). Ex vivo coating of islet cell allografts with murine CTLA4/Fc promotes graft tolerance. J. Immunol. 155, 1165-1174.
23. Ting,J.T., Balsamo,R.A., Ratnayake,C., and Huang,A.H. (1997). Oleosin of plant seed oil bodies is correctly targeted to the lipid bodies in transformed yeast. J. Biol. Chem. 272, 3699-3706.
24. Vaccaro,C., Zhou,J., Ober,R.J., and Ward,E.S. (2005). Engineering the Fc region of immunoglobulin G to modulate in vivo antibody levels. Nat. Biotechnol. 23, 1283-1288.
25. van Rooijen,G.J., Terning,L.I., and Moloney,M.M. (1992). Nucleotide sequence of an Arabidopsis thaliana oleosin gene. Plant Mol. Biol. 18, 1177-1179.
26. von Bulow,G.U. and Bram,R.J. (1997). NF-AT activation induced by a CAML-interacting member of the tumor necrosis factor receptor superfamily. Science 278, 138-141.
27. Wu,Y., Bressette,D., Carrell,J.A., Kaufman,T., Feng,P., Taylor,K., Gan,Y., Cho,H. Garcia,A.D., Gollatz,E., Dimke,D., LaFleur,D., Migone,T.S., Nardelli,B., Wei,P., Ruben,S.M., Ullrich,S.J., Olsen,H.S., Kanakaraj,P., Moore,P.A., and Baker,K.P. (2000). Tumor necrosis factor (TNF) receptor superfamily member TACI is a high affinity receptor for TNF family members APRIL and BLyS. J Biol Chem. 275, 35478-85.
28. Xia,X.Z., Treanor,J., Senaldi,G., Khare,S:D., Boone,T., Kelley,M., Theill,L.E., Colombero,A., Solovyev,I., Lee,F., McCabe,S., Elliott,R., Miner,K., Hawkins,N., Guo,J., Stolina,M., Yu,G., Wang,J., Delaney,J., Meng,S.Y., Boyle,W.J., and Hsu,H. (2000). TACI is a TRAF-interacting receptor for TALL-1, a tumor necrosis factor family member involved in B cell regulation. J. Exp. Med. 192, 137-143.

## Claims

1. A method for purifying a TACI-Fc protein comprising the steps of:
a) mixing a solution comprising protein A oilbodies with a sample comprising said TACI-Fc protein in such a way as to obtain a final ratio of mg of TACI-Fc protein per mg of dry-weight oilbodies within a range of 1 to 5.5; and
b) separating said protein A oilbodies from said TACI-Fc protein by adding an aqueous solution having a pH comprised within a range of 3.5 to 3.9,
wherein the solution comprising TACI-Fc obtained at the end of step (b) comprises more than 93%, 94%, 95% or 96% of TACI-Fc dimers.

2. The method of claim 1, wherein said TACI-Fc protein has a sequence having at least 90 %, at least 95 % or at least 99% identity to SEQ ID NO: 2, preferably wherein said TACI-Fc protein has the sequence of SEQ ID NO: 2.

3. The method of any of the preceding claims, further comprising the step of washing said protein A oilbodies prior to performing step (a), and/or further comprising the step of washing said protein A oilbodies between step (a) and (b).

4. The method of claim 3, wherein the protein A oilbodies are washed with a phosphate buffer at a pH of 7.

5. The method of any claims 3 or 4, wherein said step of washing said protein A oilbodies is repeated two or three times.

6. The method of any of the preceding claims, wherein said final ratio of mg of TACI-Fc protein per mg of dry-weight oilbodies in step (a) is within a range of 3.5 to 5.

7. The method of any of the preceding claims, wherein step (b) is performed by adding an aqueous solution having a pH of 3.5.

8. The method of claim 7, wherein said aqueous solution comprises 200 mM acetate.

9. The method of any of the preceding claims, wherein step (b) is repeated two or three times.

10. The method of any of the preceding claims, further comprising a step (c) of filtering the solution comprising said TACI-Fc protein obtained at the end of step (b).

11. The method of any of the preceding claims, further comprising a step (d) of neutralizing the solution obtained at the end of step (c).

12. The method of any of the preceding claims, further comprising one or more polishing steps.

13. The method of any of the preceding claims, wherein said TACI-Fc protein is further formulated into a pharmaceutical composition.

## Patentansprüche

1. Verfahren zur Aufreinigung eines TACI-Fc-Proteins umfassend die Schritte:
a) Mischen einer Lösung umfassend Ölkörper von Protein A mit einer Probe umfassend das TACI-Fc-Protein in solcher Weise, dass ein End-Verhältnis von mg an TACI-Fc-Protein pro mg an Trockengewicht Ölkörpern innerhalb eines Bereichs von 1 bis 5,5 erhalten wird;
und
b) Trennen der Ölkörper von Protein A von dem TACI-Fc-Protein durch Zugabe einer wässrigen Lösung mit einem pH-Wert, der innerhalb eines Bereichs von 3,5 bis 3,9 umfasst ist,
wobei die Lösung umfassend TACI-Fc, die am Ende von Schritt (b) erhalten wird, mehr als 93%, 94%, 95% oder 96% an TACI-Fc-Dimeren umfasst.

2. Verfahren nach Anspruch 1, wobei das TACI-Fc-Protein eine Sequenz mit mindestens 90%, mindestens 95% oder mindestens 99% Identität zu SEQ ID NR: 2 aufweist, bevorzugt wobei das TACI-Fc-Protein die Sequenz von SEQ ID NR: 2 aufweist.

3. Verfahren nach einem der vorhergehenden Ansprüche weiter umfassend den Schritt Waschen der Ölkörper von Protein A vor dem Durchführen von Schritt (a) und/oder weiter umfassend den Schritt Waschen der Ölkörper von Protein A zwischen Schritt (a) und (b).

4. Verfahren nach Anspruch 3, wobei die Ölkörper von Protein A mit einem PhosphatPuffer bei einem pH-Wert von 7 gewaschen werden.

5. Verfahren nach einem der Ansprüche 3 oder 4, wobei der Schritt Waschen der Ölkörper von Protein A zwei- oder dreimal wiederholt wird.

6. Verfahren nach einem der vorhergehenden Ansprüche, wobei das End-Verhältnis von mg an TACI-Fc-Protein pro mg an Trockengewicht Ölkörpern in Schritt (a) innerhalb eines Bereichs von 3,5 bis 5 ist.

7. Verfahren nach einem der vorhergehenden Ansprüche, wobei Schritt (b) durch Zugeben einer wässrigen Lösung mit einem pH-Wert von 3,5 durchgeführt wird.

8. Verfahren nach Anspruch 7, wobei die wässrige Lösung 200 mM Acetat umfasst.

9. Verfahren nach einem der vorhergehenden Ansprüche, wobei Schritt (b) zwei- oder dreimal wiederholt wird.

10. Verfahren nach einem der vorhergehenden Ansprüche weiter umfassend einen Schritt (c) Filtern der Lösung umfassend das TACI-Fc-Protein, die am Ende von Schritt (b) erhalten wird.

11. Verfahren nach einem der vorhergehenden Ansprüche weiter umfassend einen Schritt (d) Neutralisieren der Lösung, die am Ende von Schritt (c) erhalten wird.

12. Verfahren nach einem der vorhergehenden Ansprüche weiter umfassend einen oder mehrere Polierschritte.

13. Verfahren nach einem der vorhergehenden Ansprüche, wobei das TACI-Fc-Protein weiter in eine pharmazeutische Zusammensetzung formuliert wird.

## Revendications

1. Procédé de purification d'une protéine TACI-Fc, comprenant les étapes suivantes :
a) mélanger une solution comprenant des corps huileux associés à une protéine A avec un échantillon comprenant ladite protéine TACI-Fc, de manière à obtenir un rapport final du poids en milligrammes de protéine TACI-Fc au poids à sec en milligrammes des corps huileux situé dans l'intervalle allant de 1 à 5,5;
et
b) séparer lesdits corps huileux associés à une protéine A d'avec ladite protéine TACI-Fc, en ajoutant une solution aqueuse dont le pH vaut de 3,5 à 3,9,
dans lequel procédé la solution comprenant de la protéine TACI-Fc qu'on obtient à la fin de l'étape (b) contient plus de 93 %, 94 %, 95 % ou 96 % de dimères de TACI-Fc.

2. Procédé conforme à la revendication 1, dans lequel ladite protéine TACI-Fc présente une séquence identique, pour au moins 90 %, au moins 95 % ou au moins 99 %, à la Séquence N° 2, et de préférence, ladite protéine TACI-Fc présente la séquence donnée en tant que Séquence N° 2.

3. Procédé conforme à l'une des revendications précédentes, qui comprend en outre une étape de lavage desdits corps huileux associés à une protéine A, effectuée avant l'étape (a), et/ou qui comprend en outre une étape de lavage desdits corps huileux associés à une protéine A, effectuée entre l'étape (a) et l'étape (b).

4. Procédé conforme à la revendication 3, dans lequel les corps huileux associés à une protéine A sont lavés avec une solution tampon de phosphate dont le pH vaut 7.

5. Procédé conforme à l'une des revendications 3 et 4, dans lequel ladite étape de lavage desdits corps huileux associés à une protéine A est répétée à deux ou trois reprises.

6. Procédé conforme à l'une des revendications précédentes, dans lequel, dans l'étape (b), ledit rapport final du poids en milligrammes de protéine TACI-Fc au poids à sec en milligrammes des corps huileux se situe dans l'intervalle allant de 3,5 à 5.

7. Procédé conforme à l'une des revendications précédentes, dans lequel on effectue l'étape (b) en ajoutant une solution aqueuse dont le pH vaut 3,5.

8. Procédé conforme à la revendication 7, dans lequel ladite solution aqueuse contient 200 mM d'acétate.

9. Procédé conforme à l'une des revendications précédentes, dans lequel l'étape (b) est répétée à deux ou trois reprises.

10. Procédé conforme à l'une des revendications précédentes, qui comporte en outre une étape (c) consistant à filtrer ladite solution comprenant de la protéine TACI-Fc, obtenue à la fin de l'étape (b).

11. Procédé conforme à l'une des revendications précédentes, qui comporte en outre une étape (d) consistant à neutraliser la solution obtenue à la fin de l'étape (c).

12. Procédé conforme à l'une des revendications précédentes, qui comporte en outre une ou plusieurs étape(s) de polissage.

13. Procédé conforme à l'une des revendications précédentes, dans lequel ladite protéine TACI-Fc est ensuite introduite en formulation dans une composition pharmaceutique.


### SEQUENCE LISTING

<110> LABORATOIRES SERONO S.A.
<120> PURIFICATION OF Fc-FUSION PROTEINS USING THE OILBODY TECHNOLOGY
<130> 1156 WO/PCT
<150> EP 07101233.0
   <151> 2007-01-26
<150> 60/8886,681
   <151> 2007-01-26
<160> 4
<170> PatentIn version 3.1
<210> 1
   <211> 293
   <212> PRT
   <213> Homo sapiens
<400> 1
<210> 2
   <211> 348
   <212> PRT
   <213> Artificial
<220>
   <223> TACI-Fc fusion protein
<400> 2
   Met Asp Ala Met Lys Arg Gly Leu Cys Cys Val Leu Leu Leu Cys Gly
   1               5                   10                  15
   Ala Val Phe Val Ser Leu Ser Gln Glu Ile His Ala Glu Leu Arg Arg
               20                  25                  30
   Phe Arg Arg Ala Met Arg Ser Cys Pro Glu Glu Gln Tyr Trp Asp Pro
           35                  40                  45
   Leu Leu Gly Thr Cys Met Ser Cys Lys Thr Ile Cys Asn His Gln Ser
       50                  55                  60
   Gln Arg Thr Cys Ala Ala Phe Cys Arg Ser Leu Ser Cys Arg Lys Glu
   65                  70                  75                  80
   Gln Gly Lys Phe Tyr Asp His Leu Leu Arg Asp Cys Ile Ser Cys Ala
                   85                  90                  95
   Ser Ile Cys Gly Gln His Pro Lys Gln Cys Ala Tyr Phe Cys Glu Asn
               100                 105                 110
   Lys Leu Arg Ser Glu Pro Lys Ser Ser Asp Lys Thr His Thr Cys Pro
           115                 120                 125
   Pro Cys Pro Ala Pro Glu Ala Glu Gly Ala Pro Ser Val Phe Leu Phe
       130                 135                 140
   Pro Pro Lys Pro Lys Asp Thr Leu Met Ile Ser Arg Thr Pro Glu Val
   145                 150                 155                 160
   Thr Cys Val Val Val Asp Val Ser His Glu Asp Pro Glu Val Lys Phe
                   165                 170                 175
   Asn Trp Tyr Val Asp Gly Val Glu Val His Asn Ala Lys Thr Lys Pro
               180                 185                 190
   Arg Glu Glu Gln Tyr Asn Ser Thr Tyr Arg Val Val Ser Val Leu Thr
           195                 200                 205
   Val Leu His Gln Asp Trp Leu Asn Gly Lys Glu Tyr Lys Cys Lys Val
       210                 215                 220
   Ser Asn Lys Ala Leu Pro Ser Ser Ile Glu Lys Thr Ile Ser Lys Ala
   225                 230                 235                 240
   Lys Gly Gln Pro Arg Glu Pro Gln Val Tyr Thr Leu Pro Pro Ser Arg
                   245                 250                 255
   Asp Glu Leu Thr Lys Asn Gln Val Ser Leu Thr Cys Leu Val Lys Gly
               260                 265                 270
   Phe Tyr Pro Ser Asp Ile Ala Val Glu Trp Glu Ser Asn Gly Gln Pro
           275                 280                 285
   Glu Asn Asn Tyr Lys Thr Thr Pro Pro Val Leu Asp Ser Asp Gly Ser
       290                 295                 300
   Phe Phe Leu Tyr Ser Lys Leu Thr Val Asp Lys Ser Arg Trp Gln Gln
   305                 310                 315                 320
   Gly Asn Val Phe Ser Cys Ser Val Met His Glu Ala Leu His Asn His
                   325                 330                 335
   Tyr Thr Gln Lys Ser Leu Ser Leu Ser Pro Gly Lys
               340                 345
<210> 3
   <211> 1044
   <212> DNA
   <213> Artificial
<220>
   <223> Polynuceotide encoding a TACI-Fc fusion protein
<400> 3
   atggatgcaa tgaagagagg gctctgctgt gtgctgctgc tgtgtggcgc cgtcttcgtt 60
   tcgctcagcc aggaaatcca tgccgagttg agacgcttcc gtagagctat gagatcctgc 120
   cccgaagagc agtactggga tcctctgctg ggtacctgca tgtcctgcaa aaccatttgc 180
   aaccatcaga gccagcgcac ctgtgcagcc ttctgcaggt cactcagctg ccgcaaggag 240
   caaggcaagt tctatgacca tctcctgagg gactgcatca gctgtgcctc catctgtgga 300
   cagcacccta agcaatgtgc atacttctgt gagaacaagc tcaggagcga gcccaaatct    360
   tcagacaaaa ctcacacatg cccaccgtgc ccagcacctg aagccgaggg ggcaccgtca    420
   gtcttcctct tccccccaaa acccaaggac accctcatga tctcccggac ccctgaggtc    480
   acatgcgtgg tggtggacgt gagccacgaa gaccctgagg tcaagttcaa ctggtacgtg    540
   gacggcgtgg aggtgcataa tgccaagaca aagccgcggg aggagcagta caacagcacg    600
   taccgtgtgg tcagcgtcct caccgtcctg caccaggact ggctgaatgg caaggagtac    660
   aagtgcaagg tctccaacaa agccctccca tcctccatcg agaaaaccat ctccaaagcc    720
   aaagggcagc cccgagaacc acaggtgtac accctgcccc catcccggga tgagctgacc    780
   aagaaccagg tcagcctgac ctgcctggtc aaaggcttct atcccagcga catcgccgtg    840
   gagtgggaga gcaatgggca gccggagaac aactacaaga ccacgcctcc cgtgctggac    900
   tccgacggct ccttcttcct ctacagcaag ctcaccgtgg acaagagcag gtggcagcag    960
   gggaacgtct tctcatgctc cgtgatgcat gaggctctgc acaaccacta cacgcagaag   1020
   agcctctccc tgtctccggg taaa                                          1044
<210> 4
   <211> 324
   <212> PRT
   <213> Artificial
<220>
   <223> Oleosin-protein A fusion protein
<400> 4
   Tyr Ala Thr Gly Glu His Pro Gln Gly Ser Asp Lys Leu Asp Ser Ala
   1               5                   10                  15
   Arg Met Lys Leu Gly Ser Lys Ala Gln Asp Leu Lys Asp Arg Ala Gln
               20                  25                  30
   Tyr Tyr Gly Gln Gln His Thr Gly Gly Glu His Asp Arg Asp Arg Thr
           35                  40                  45
   Arg Gly Gly Gln His Thr Thr Leu Val Pro Arg Gly Ser Met Asp Gln
       50                  55                  60
   Arg Asn Gly Phe Ile Gln Ser Leu Lys Asp Asp Pro Ser Gln Ser Ala
   65                  70                  75                  80
   Asn Val Leu Gly Glu Ala Gln Lys Leu Asn Asp Ser Gln Ala Pro Lys
   85 90 95
   Ala Asp Ala Gln Gln Asn Asn Phe Asn Lys Asp Gln Gln Ser Ala Phe
   100 105 110
   Tyr Glu Ile Leu Asn Met Pro Asn Leu Asn Glu Ala Gln Arg Asn Gly
   115 120 125
   Phe Ile Gln Ser Leu Lys Asp Asp Pro Ser Gln Ser Thr Asn Val Leu
   130 135 140
   Gly Glu Ala Lys Lys Leu Asn Glu Ser Gln Ala Pro Lys Ala Asp Asn
   145 150 155 160
   Asn Phe Asn Lys Glu Gln Gln Asn Ala Phe Tyr Glu Ile Leu Asn Met
   165 170 175
   Pro Asn Leu Asn Glu Glu Gln Arg Asn Gly Phe Ile Gln Ser Leu Lys
   180 185 190
   Asp Asp Pro Ser Gln Ser Ala Asn Leu Leu Ser Glu Ala Lys Lys Leu
   195 200 205
   Asn Glu Ser Gln Ala Pro Lys Ala Asp Asn Lys Phe Asn Lys Glu Gln
   210 215 220
   Gln Asn Ala Phe Tyr Glu Ile Leu His Leu Pro Asn Leu Asn Glu Glu
   225 230 235 240
   Gln Arg Asn Gly Phe Ile Gln Ser Leu Lys Asp Asp Pro Ser Gln Ser
   245 250 255
   Ala Asn Leu Leu Ala Glu Ala Lys Lys Leu Asn Asp Ala Gln Ala Pro
   260 265 270
   Lys Ala Asp Asn Lys Phe Asn Lys Glu Gln Gln Asn Ala Phe Tyr Glu
   275 280 285
   Ile Leu His Leu Pro Asn Leu Thr Glu Glu Gln Arg Asn Gly Phe Ile
       290                 295                 300
   Gln Ser Leu Lys Asp Asp Pro Gly Asn Ser Arg Gly Ser Val Asp Leu
   305                 310                 315                 320
   Gln Ile Thr Asn